# EUROPEAN PATENT SPECIFICATION

(11) **EP 1 722 684 B1**
(45) Date of publication and mention of the grant of the patent: **10.09.2008**
(21) Application number: 05711153.6
(22) Date of filing: 02.03.2005
(51) Int. Cl.: A61B 5/155, G01N 33/487

(54) **SYSTEM AND METHOD FOR AUTOMATIC TAKING OF FLUID SAMPLES**
SYSTEM UND VERFAHREN FÜR DIE AUTOMATISCHE ENTNAHME VON FLÜSSIGKEITSPROBEN
SYSTEME ET PROCEDE DE PRELEVEMENT AUTOMATIQUE D'ECHANTILLONS DE FLUIDE

(30) Priority: 05.03.2004 SE 0400561; 08.03.2004 US 521193 P
(43) Date of publication of application: 22.11.2006
(73) Proprietor: DiLab i Lund AB, 226 42 Lund (SE)
(72) Inventor: HANSSON, Hans-Axel, S-224 65 Lund (SE)
(74) Representative: Akerman, Marten Lennart
(86) International application number: PCT/SE2005/000295
(87) International publication number: WO 2005/084547

(56) References cited:
- EP-A2- 0 256 415
- EP-A2- 0 513 789
- WO-A1-01/78591
- WO-A1-83/03057
- WO-A1-03/032834
- US-A- 3 600 953

## Description

### Technical Field

The present invention relates generally to automatic taking of fluid samples from a test object in the shape of a living being. More specifically, the invention relates to automatic taking of fluid samples having a composition representative of the composition of the fluid at the sample site of the living being.

### Background

In medical science and pharmaceutical industry there is often a need to perform pharmacokinetic studies on living beings, for example test objects in the shape of laboratory animals such as rats and pigs. In such studies, it is common to take a plurality of samples or specimens from the test object as well as injecting substances into the test object during the course of hours or days, in order to allow observation of gradual responses in the test object. In order to minimise time and cost consuming manual handling of taking specimen as well as the stress impact of such a manual handling on the laboratory animal, attempts have been made to automate the sample taking procedure.

In prior art there are automatic systems for taking of specimens from and for delivering injections to living beings, for example laboratory animals used for experimental purposes such as rats and pigs. A drawback with some of the prior art system is that the volume of a taken sample usually is much larger than the volume necessary for the analysis of a taken sample. The reason for withdrawing a much larger volume of the body fluid than the volume than is used for the analysis is to obtain a sample that is to be analysed that has a composition representative of the composition of the body fluid in the test object. Many systems of today utilises tubings comprising a rinsing fluid or the like and when a taken sample is introduced into the tubings it will be diluted due to the cross-mixing between the taken sample and the rinsing fluid. Consequently, the taken sample will not show a composition that is representative of the body fluid. Thus, if the taken sample is to be analysed, the analysis would not give a representative result.

The unnecessary large volume that has to be taken is especially a drawback when taking blood samples from test objects, e.g. rats and mice, having a small total blood volume. At taking of blood sample from rats or mice, it is only possible to take a blood volume that corresponds to approximately 1-2 weight-% of the animal without negatively affecting the state of health of the living being. If larger sample volumes are taken, one has to interrupt the sample taking procedure for several days in order to let the test object recover before a new sample can be taken.

When using some of the systems available today, one has withdraw a blood volume of approximately 200 micro litres from the test object in order to obtain a taken sample having a low percentage of dilution. The system of today results in 10% dilution of a taken sample and in that only approximately 10 samples can be taken from a the test object, e.g. a rat, in order not to exceed the total volume of 2-3 millilitres that can be taken from the rat. Thus in repeated taking of blood samples it is desirable to minimise the total blood volume that is taken from the test object and that the entire or almost the entire volume of a taken sample is used for analysis. The latter is possible only if the taken blood sample has a low percentage of dilution.

In many pharmacological studies, it is also desirable to obtain more sample and samples having a smaller volume, which neither are possible with the automatic systems of today.

The prior art document US 4,691,580 to Fosslien shows a fluid sample apparatus wherein an air bubble is used to reduce cross-mixing of saline wash with blood samples and to separate blood samples.

The prior art document EP 0 389 719 to International Technidyne Corp shows an example of a sample collection and delivery system adapted for body fluid sample. The samples are transported in a lumen of a tubing from one end to other with a separation between each sample. A drawback with this system is that one or several samples (reference numeral 62 in EP 0 389 719) is taken prior to the sample (reference numeral 63 in EP 0 389 719) that is to be analysed in order to collect any residual of washing fluid that may remain in contact with the inner surface of the sample transfer tube. Thus, a larger volume of the sample is taken than is required for the analysis. This is especially critically when the samples are taken from a living being having a small total volume of the sample fluid. Thus fewer samples can be taken than if the volume required to be taken per analysed sample where less. Another drawback with the system is that the disclosed washing procedure (Fig. 2K in EP 0 389 719) causes all of the washing fluid flow to be directed through the catheter into the vessel of the living being. This supply of washing fluid to the vessel causes a dilution of the blood. If a new blood sample is taken its composition would not be the same as if the washing fluid was not supplied to the blood vessel. Thus in order to take samples having a representative composition, one has to wait for a quite long time before taking another sample to be analysed. Another drawback with supplying washing fluid to the vessel is that it changes the fluid balance of the living being. Yet another drawback with supplying washing fluid to the vessel is that the washing fluid usually comprises heparin in order to prevent coagulation of blood in the catheter means but can be harmful to supply to the living being.

The prior art document WO 03/032834 discloses a method for sampling body fluid of a living being, a sample is led via a catheter, canola or the like, which is closed with a shutter, after which at least one transport medium is led into a tube behind the sample.

### Object of the Invention

The overall object of the present invention is to solve the problem of taking a fluid sample of a body fluid having the same composition as the body fluid at the sample site of the test object. An aspect of the object is to provide an automatic system for taking a fluid sample from a test object that minimises the dilution of the taken sample.

A more specific object of the present invention is to provide a blood sample having a composition corresponding to the composition of blood in the normal blood flow at the sample site of a test object. An aspect of this object is to provide an automatic system for taking blood samples from a test object that minimises the dilution of the taken blood sample.

By means of the present invention, several advantages are achieved as compared to the prior art. Amongst others, a taken fluid sample having a higher concentration as compared to a fluid sample taken by a prior art system is achieved. Another advantage is that a minimal amount of rinsing solution is supplied to the sample site reducing the dilution of the body fluid as compared to the prior art systems. Yet another advantage is that it provides an improved procedure for washing the lumens of the catheter means, whereby a drug solution can be supplied to the sample site by means of the same catheter means that is used for taking the sample with a minimised risk of contamination.

### Summary of the invention

The above-mentioned objects and aspects, amongst others, are obtained by the present invention. The invention refers to a system, a method and a computer program product according to the independent claims. Preferred embodiments of the invention are specified in the dependent claims.

### Brief description of the drawings

The present invention will be described in more detail with reference to the accompanying drawings, in which:
FIG. 1 schematically shows an embodiment of the inventive system;
FIGS. 2A - 2I schematically show parts of the embodiment of the inventive system of FIG. 1 and the steps of the sample taking procedure provided by the invention; and
FIG. 3 schematically shows an embodiment of a double lumen catheter means for use in the inventive system.

### Detailed description of the invention

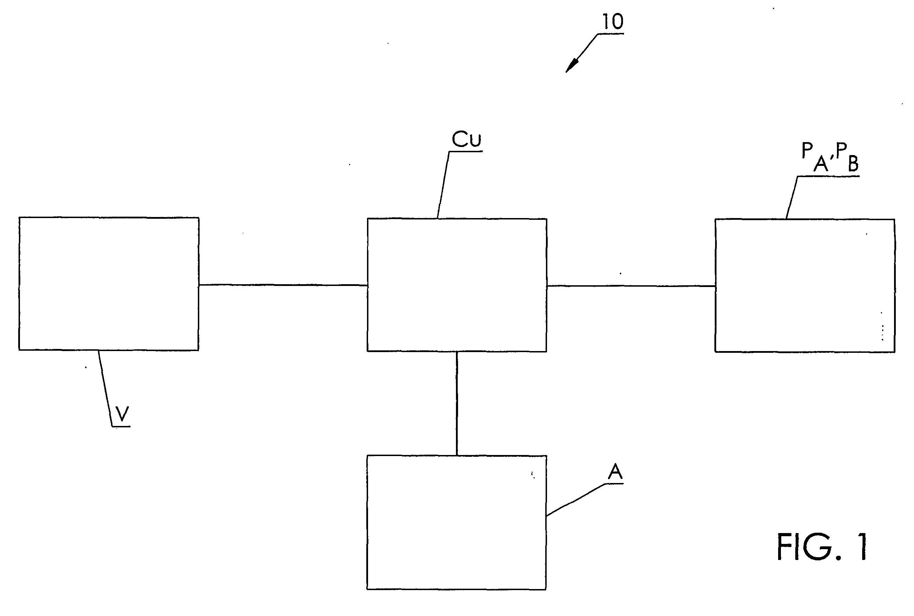

The present invention will now be described in more detail with reference to the accompanying drawings. FIG. 1 shows an embodiment of a system for automatic taking of specimens according to the invention. The figure schematically illustrates a control unit CU communicatively connected to pumping means P_{A}, P_{B} and a set of valves V. The control unit CU is configured to control the operation of the pumping means P_{A}, P_{B} and the set of valves V. The pumping means can for example be realised as a piston pump or a reciprocating pump. The system 10 according to the figure also comprises an analyser A communicatively connected to the control unit CU. However, it should be understood that the analyser can be a separate unit not comprised in the system 10 but instead connected to the system 10. The analyser A is configured to analyse a taken sample.

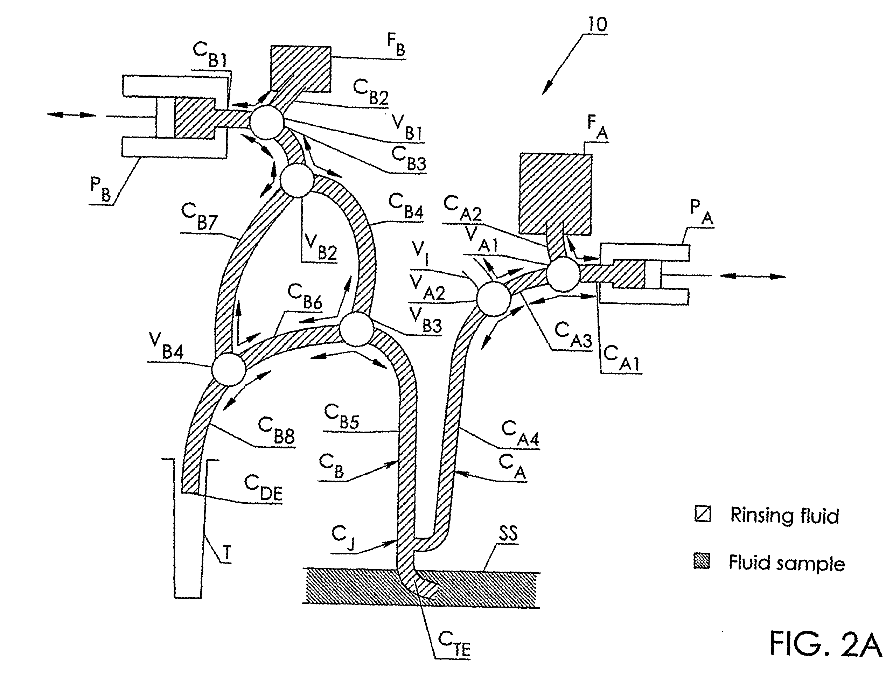

As shown in the FIGS. 2A-2I, the system 10 comprises also flexible tubings, e.g. first and second catheter means C_{A} and C_{B}, respectively. In the shown embodiment, the system 10 comprises further a plurality of valves V_{A1}, V_{A2}, V_{B1}, V_{B2}, V_{B3}, V_{B4} connected to the catheter means C_{A}, C_{B} and a first and second source of rinsing/washing fluid F_{A} and F_{B}, respectively. The first and second source of rinsing fluid F_{A} and F_{B} being arranged at valves V_{A1} and V_{B1}, respectively. It should be understood that the first and second rinsing fluid sources can be arranged as one single source having two outlet openings, each connectable to a valve in catheter means C_{A} and C_{B}, respectively.

The catheter means C_{A} and C_{B} comprises each a lumen. Further, several catheter parts or tubings can constitute the catheter means C_{A} and C_{B}. In the embodiment shown in FIGS. 2A-2I, the first catheter means C_{A} comprises a first part C_{A1} connecting the first pumping means P_{A} and a first valve V_{A1}, and a second part C_{A2} connecting the first fluid source F_{A} and the first valve VA_{1.} The catheter means C_{A} comprises further a third part C_{A3} connecting the first valve V_{A1} and a second valve V_{A2}, and a fourth part C_{A4} connecting the second valve V_{A2} and a three-way junction C_{J}.

As also shown in FIG. 2A, the second catheter means C_{B} comprises a first part C_{B1} connecting the second pumping means P_{B} and a first valve V_{B1}, and a second part C_{B2} connecting the second fluid source F_{B} and the first valve V_{B1}. The catheter means C_{B} comprises further a third part C_{B3} connecting the first valve V_{B1} and a second valve V_{B2}, and a fourth part C_{B4} connecting the second valve V_{B2} and a third valve V_{B3}. A fifth part C_{B5} is arranged to connect the third valve V_{B3} and the three-way junction C_{J}, a sixth part C_{B6} connects the third valve V_{B3} and a fourth valve V_{B4}. A seventh part C_{B7} is arranged to connect the second and the fourth valves V_{B2} and V_{B4}, and an eighth part C_{B8} extends from the fourth valve V_{B4} to a sample tube T.

The valves are configured to control the flow path of the flow of a fluid in the catheter means and the pumping means are configured to control the flow rate and the flow direction of the fluid comprised in the catheter means.

It should be understood that the parts of the catheter means are only schematically illustrated in the FIGS. 2A-2I, e.g. are the dimensions, i.e. the diameter and the length, of the different parts not according to scale and serve only to exemplify principles of the invention.

The two-way arrows at each of the valves in FIG. 2A, indicate the possible flow directions provided by each of the plurality of valves comprised in the system, i.e. the possible flow paths of the fluid through the respective valve.

An embodiment of the inventive method for automatic taking of a specimen will now be described with reference to FIG. 2A - 2I.

In step 100 a rinsing fluid, e.g. a heparinised sodium chloride solution, has been supplied to i.a. the lumens of the tubings or catheter means C_{A}, C_{B} and possibly also to the pumping means P_{A}, P_{B} of the system 10, cf. FIG. 2A.

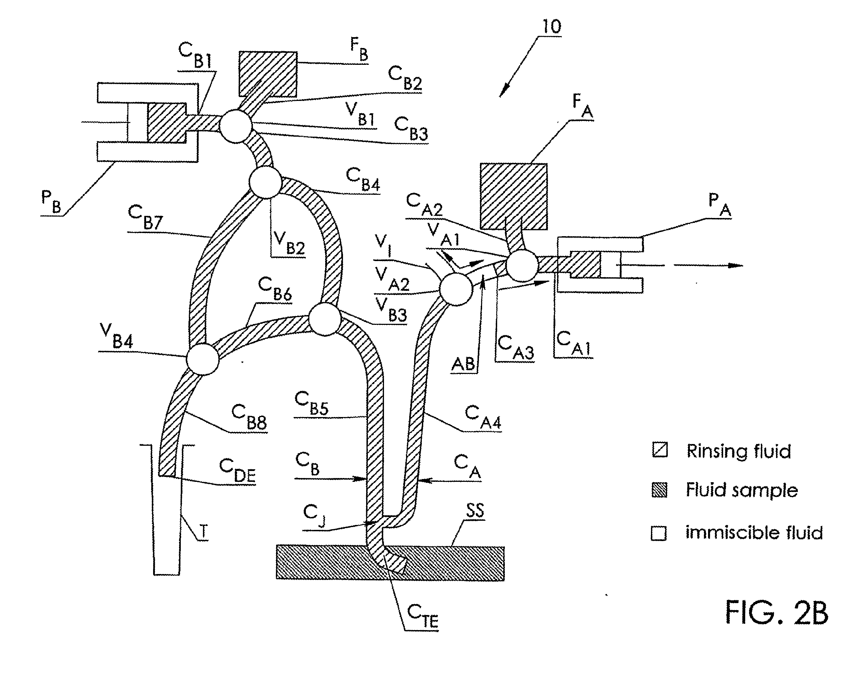

In step 102, an inlet V_{I} of the valve A2 is opened and the pumping means P_{A} provides an suction action, whereby an amount of an immiscible fluid, e.g. an air bubble, AB is sucked into the lumen of the catheter means C_{A3}, cf. FIG. 2B. The flow direction and the operating direction of the first pumping means P_{A} is indicated by the arrows. The second pumping means P_{B} is not active.

In step 104, the air bubble AB is moved in the catheter means C_{A} towards the sample site SS, e.g. a blood vessel, of the test object. The movement of the air bubble AB and the rinsing solution in the catheter means C_{A}, C_{B} is accomplished by the pumping means P_{A}, P_{B}. The control unit CU is configured to control the pumping means P_{A}, P_{B} to transport said immiscible fluid AB to the three-way junction C_{J} of the catheter means C_{A}, C_{B}. A first part AB₁ of said immiscible fluid AB will preferably be located in the fifth catheter part C_{B5} of the second catheter means C_{B} and a second part AB₂ of said immiscible fluid AB will be located in the fourth catheter part C_{A4} of the first catheter means C_{A}. The first AB₁ and second AB₂ parts of the immiscible fluid AB is approximately of the same volume.

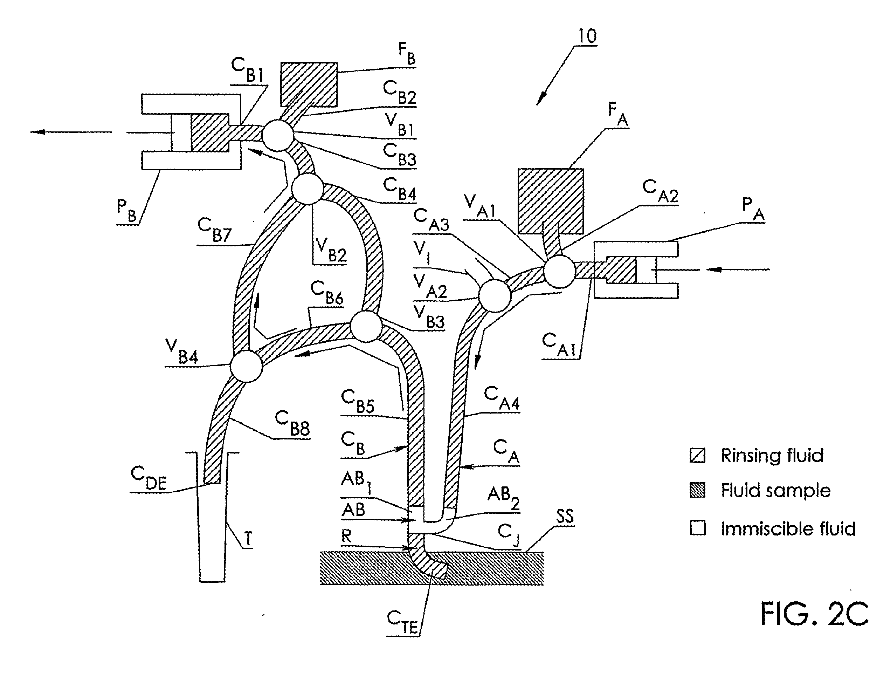

In order to accomplish the movement of the air bubble AB towards the three-way junction C_{J}, the control unit CU is preferably arranged to control the first pumping means P_{A} to provide a pushing action and the second pumping means P_{B} to provide a suction action. The pushing and suction actions being of the same size or almost of the same size. In other words the first and second pumping means P_{A}, P_{B} are preferably controlled to provide the same flow rate but in opposite directions. The arrows in FIG. 2C, indicate the flow direction of the rinsing fluid and the air bubble AB, and the operating directions of the pumping means P_{A} P_{B}. FIG. 2C schematically shows the situation when the air bubble AB has been moved to the junction C_{J}.

An advantage will the present invention is that when the immiscible fluid is transported to the junction, the rinsing fluid comprised down streams of the immiscible fluid is transported to the junction and then via the second catheter means to a waste container or the like. Thus, no rinsing fluid is supplied to the sample site during this operation. Consequently, the body fluid at the sample site is not diluted.

In step 106, the first pumping means P_{A} is turned off and the flow path through the catheter means is maintained as before. The second pumping means P_{B} provides a pushing action, whereby causing the first part AB, of the air bubble AB to move towards the sample-taking end C_{TE} of the catheter means. The second pumping means P_{B} is turned off when the first part AB₁ of the air bubble AB reaches the sample-taking end C_{TE}.

The volume R of the rinsing fluid (cf. FIG. 2C) comprised in the sample-taking end C_{TE} of the catheter means C_{A}, C_{B} is small, approximately 5 micro litres, causing a minimal dilution of the body fluid when it is supplied to the body fluid.

As mentioned above, the control unit controls the operation of the pumping means. By means of the knowledge of the volume, i.e. the length and inner diameter, of the catheter means, and the flow rate caused by the pumping means, it is possible for the control unit to determine when a desired position is reached by a part of the fluid. Thus, it is possible to determine when to stop the operation of the pumping means in order to accurately positioning the immiscible fluid at the junction. It is also possible to accurately positioning the first part of the immiscible fluid at the end opening of the sample-taking end. This is especially advantageously since the risk of introducing the first part of the immiscible fluid to the sample site is minimised. In the case the first part of the immiscible fluid is an air bubble, the risk of air embolism is eliminated.

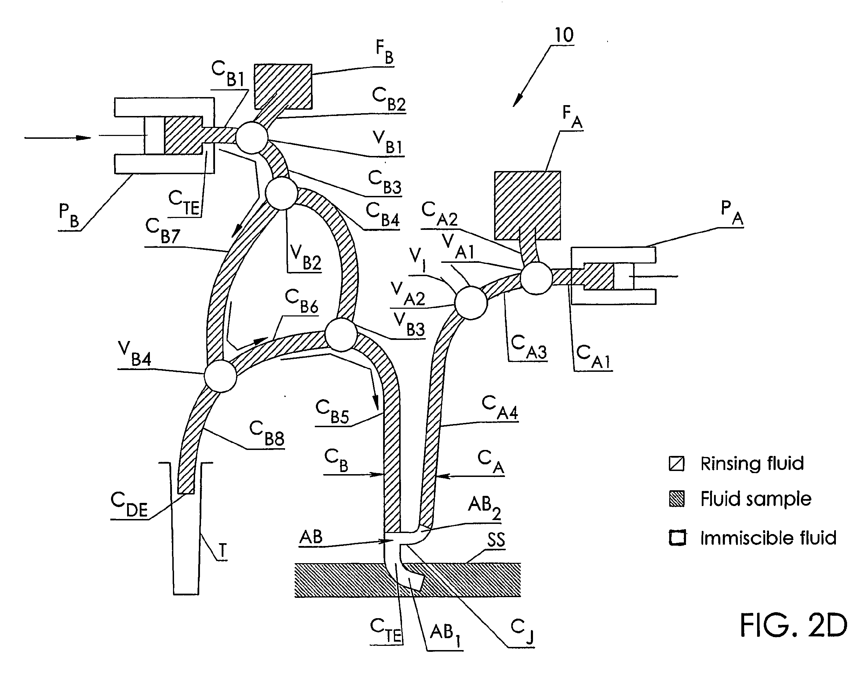

FIG. 2D schematically shows the result of step 106 and the arrows indicates the flow direction of the rinsing fluid and the first part AB₁ of the air bubble and of the operating direction of the second pumping means P_{B}.

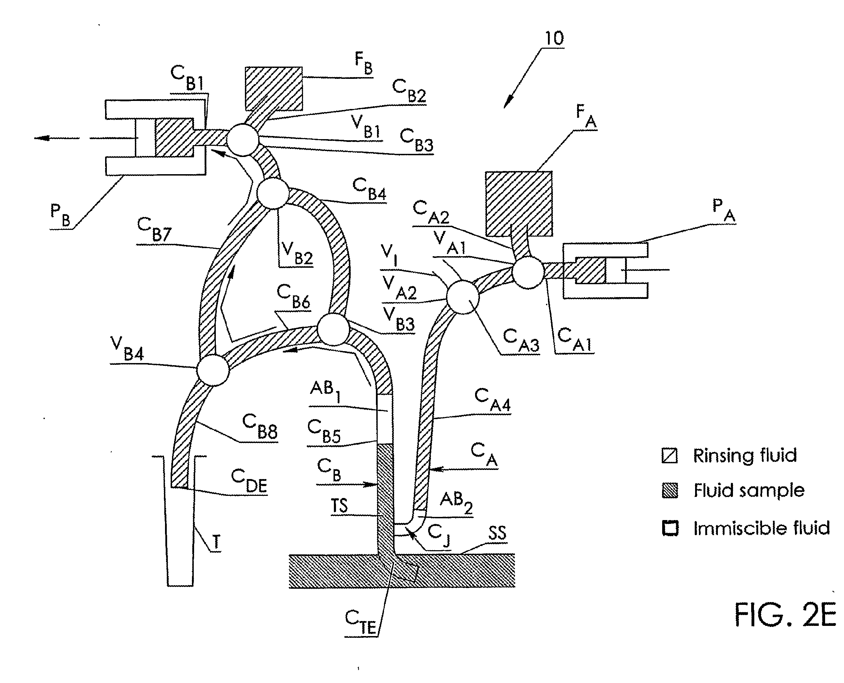

In step 108, a fluid sample is taken from the sample site SS. In the case of a blood sample, the desired sample volume is withdrawn from a blood vessel of the test object, by means of a suction action of the second pumping means P_{B}. In the catheter means, the first part AB₁ of the air bubble AB is located before the taken sample TS as indicated in FIG. 2E. During this operation, the first pumping means P_{A} is turned off. In some cases, it can be advantageously to withdraw a sample volume that is approximately five micro litres larger than the volume required for performing the analysis.

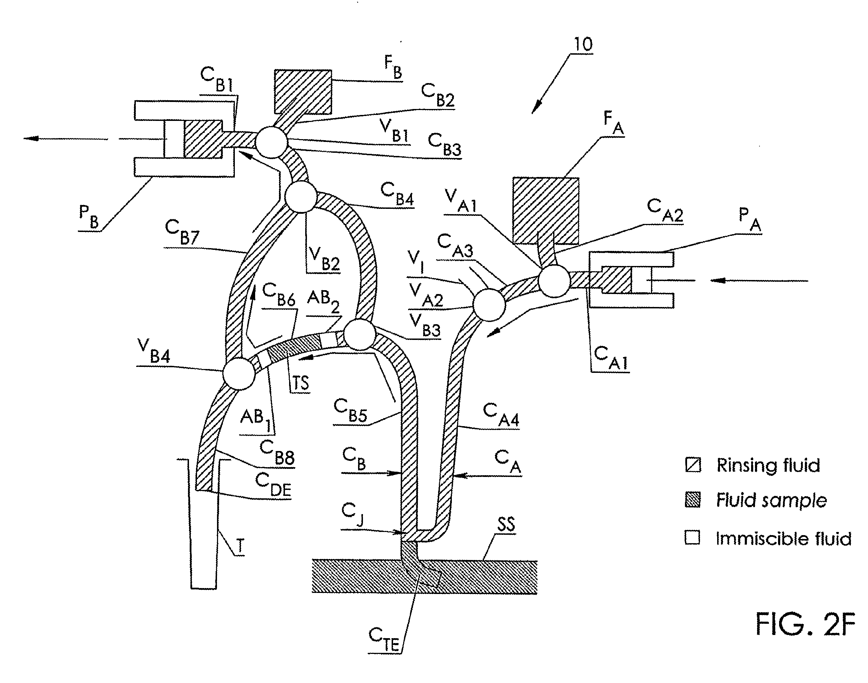

In step 110, the taken sample TS is transported towards the sample tube T by means of a suction action of the second pumping means P_{B} and a pushing action of the first pumping means P_{A}, cf. FIG. 2F. The taken sample TS and the rinsing fluid are separated by means of the first AB₁ and second AB₂ parts of the air bubble AB, respectively. This is accomplished by means of the first P_{A} and second P_{B} pumping means controlled to provided the same flow rate but in different directions, i.e. the second pumping means P_{B} provides a suction action and the first pumping means P_{A} provides a pressing action, or vice versa if the sample is to be moved in the opposite direction.

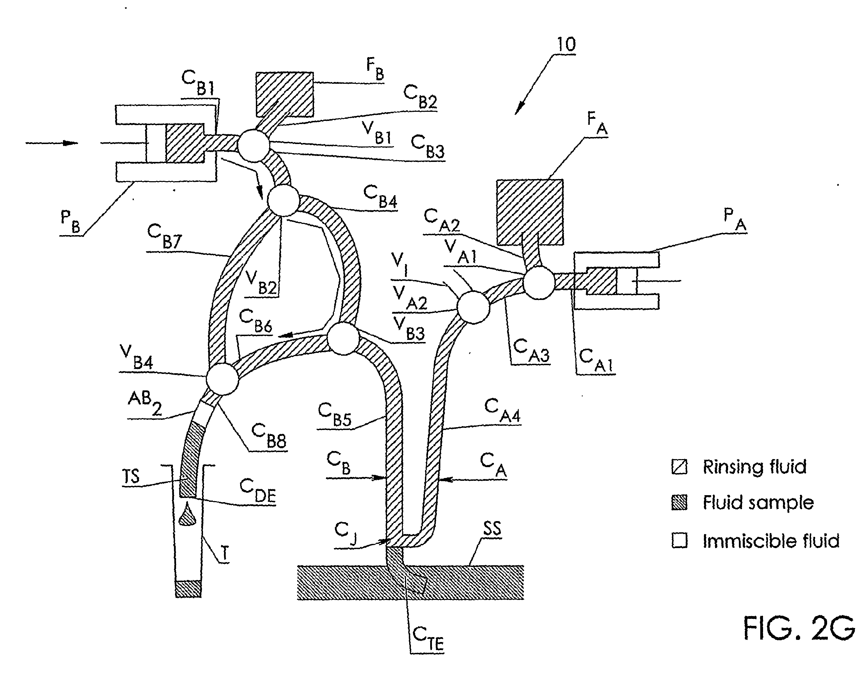

In step 112, the second pumping means P_{B} provides a pushing action, whereby the taken sample TS is transported in sixth C_{B6} and eighth C_{B8} parts of the second catheter means C_{B} towards a sample tube T arranged at a delivery end C_{DE} of the catheter means C_{B}. FIG. 2G.

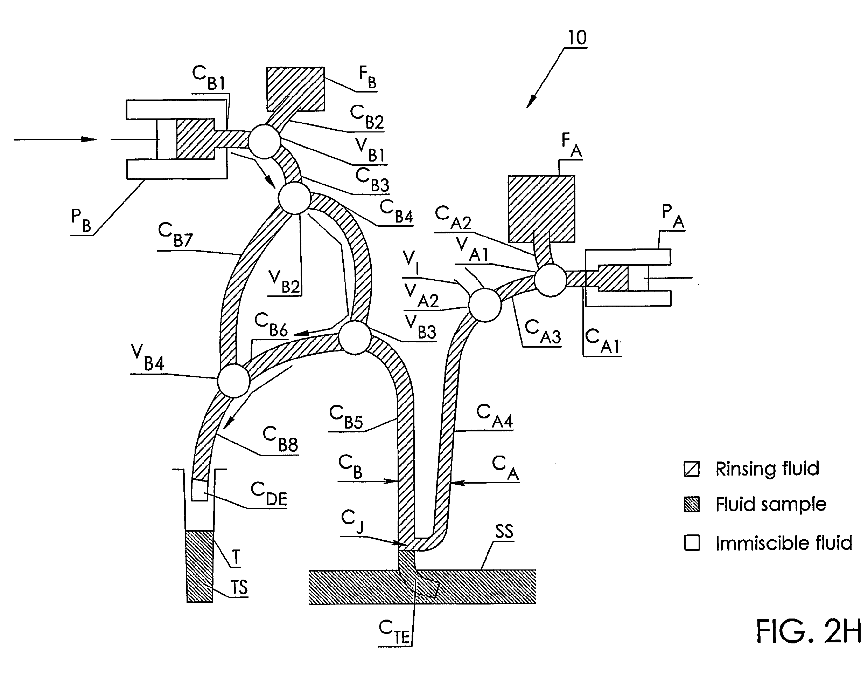

In step 114, the taken sample TS is delivered to the sample tube T, by means of the pushing action of the second pumping means P_{B}. Cf. FIG. 2H.

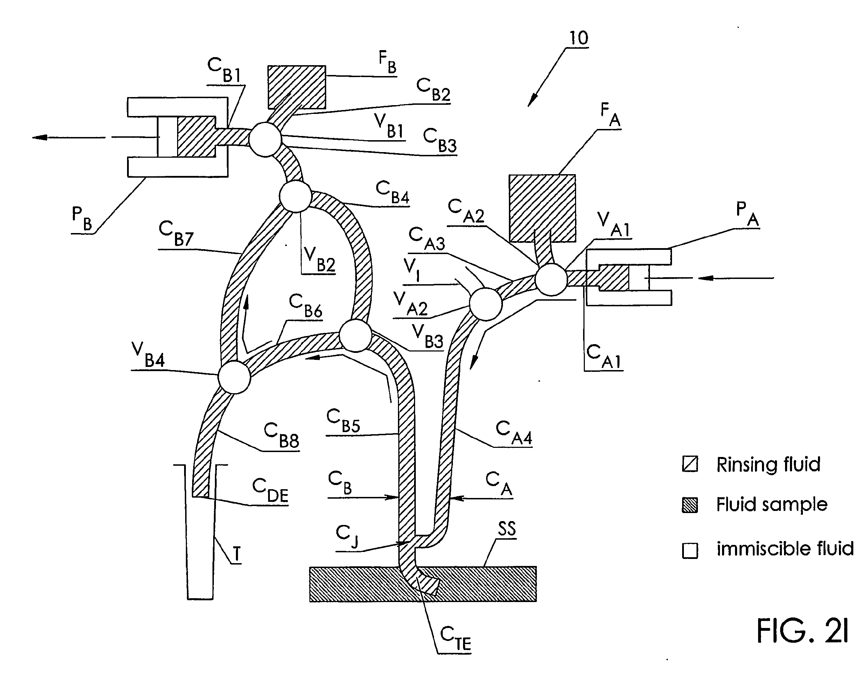

In step 116, the lumens of the first and second catheter means C_{A}, C_{B} are rinsed/washed by providing a flow of rinsing solution through the catheter means C_{A}, C_{B}. The control unit CU control the first and second pumping means P_{A} and P_{B} to operate at the same rate, whereby the catheter means are rinsed without supplying rinsing fluid to the sample site SS via the sample-taking end C_{TE} of the catheter means. Cf. FIG. 2I.

If the first and second pumping means do not operate in a synchronised manner, i.e. if for example the pushing action of the first pumping means is smaller than the suction action of the second pumping means, an unnecessary volume of sample solution can be withdrawn from the sample site. On the other hand, if the pushing action of the first pumping means is larger than the suction of the second pumping means, rinsing fluid can be supplied to the sample site. This latter case can be desirable when the sample-taking end C_{TE} of the catheter means is to be rinsed. In such case, the flow of rinsing solution is preferably accomplished by means of the first pumping means P_{A} pushing with a slightly higher pressure than the second pumping means P_{B} is sucking. In this way the part of the catheter means C_{TE} attached to the sample site also can be rinsed, without supplying any rinsing fluid to the sample site of the test object. For example, the pumping means P_{A} can be pressing at 100% of a flow F and the pumping means P_{B} can be sucking at 90% of the flow F.

It should be understood that by controlling the pumping means P_{A} and P_{B} to operate at the same rate, the catheter means can be rinsed with out risking to supply rinsing fluid to the test object. This is especially an advantage when the test object is sensitive for supply of large volume of fluid. This is for example the case when the catheter means is connected to a blood vessel of a rat or a mouse, since the supply of rinsing fluid to the blood vessel would dilute the blood and thereby change the characteristics of the normal blood and the concentration of different components in the blood. Furthermore, the supply would also changed the fluid balance of the test object.

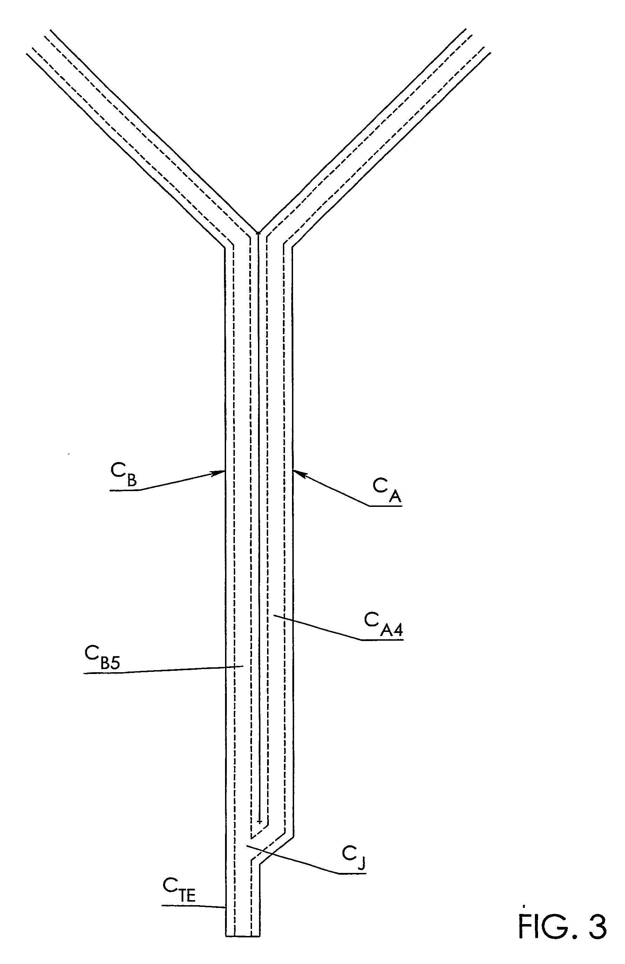

FIG. 3 schematically shows an embodiment of a double lumen catheter means C_{A}, C_{B} for using in the present invention. As shown, the catheter means comprises two lumen which at the junction coincidences to a single lumen. Further, at the end opposite to the sample-taking end, the catheter means C_{A}, C_{B} diverges into two separated catheter means C_{A} and C_{B}. Thus as described above, an aspirated air bubble can be moved from the valve inlet to the junction in the catheter part C_{A4} and the rinsing solution comprised in the catheter means downstreams the air bubble will be transported in the catheter means C_{B5} towards the junction and then via the catheter means C_{B5} to a waste container. Thus, the rinsing fluid passes by the sample-taking end without being supplied to the sample site.

The present invention has been described with reference to an embodiment of the invention. However, it should be understood that several modifications and variations of the system and method of the invention could be accomplished without falling apart from the scope of the invention. The sources of rinsing fluid, F_{A} and F_{B}, can for example be arranged as one single source of rinsing fluid. In the description text above, reference has been made to an air bubble, but it should be understood that another immiscible fluid, e.g. another gas or another suitable substance immiscible in the sample and the rinsing fluid could be used. In such cases, the immiscible fluid is for example contained in a container attached to the catheter means C_{A} at the valve V_{A2}.

The pumping means P_{A} and P_{B} can further be configured as two separated pumping means as described above but they can also be configured as one single double-acting suction and force pumping means with a first part having the capability of providing a pushing action and a second part having the capability of providing a suction action. The first and second part being configured to operate separately or at the same time. In cases having a double-acting pumping means, a third pumping means is arranged at the catheter means and configured to operate when the first and a second parts are operated separately and to compensate for the active first or second part. That is, to provide a suction action if one of the parts is providing a pushing action, and to provide a pushing action if one of the parts is providing a suction action.

Further, the invention can comprise a source of a drug solution connectable to said catheter means (C_{A}, C_{B}). Due to the inventive arrangement of the catheter means and the pumping means, a drug can be supplied to a test object via the catheter means by means of the pumping means and the catheter means can subsequently be thoroughly washed in order to remove any possible drug residuals before a sample is taken by means of the same catheter means and pumping means. Due to the advantageously way to provide a fluid flow through the catheter means, the catheter means can be thoroughly washed without supplying any rinsing fluid to the sample site.

## Claims

1. An automatic system (10) for taking of a fluid sample from a sample site (SS) of a living test object, comprising:
- catheter means (C_{A}, C_{B}) comprising a three-way junction (C_{J}) configured to be located in proximity to said sample site (SS), said three-way junction (CJ) is connected to a first catheter means (C_{A}), a second catheter means (C_{B}) and a sample-taking end (C_{TE});
- a valve (V_{A2}) connected to said first catheter means (C_{A}), said valve (V_{A2}) having an inlet (V_{I}) for an immiscible fluid to be aspirated into said first catheter means (C_{A}); and
- pumping means (P_{A}, P_{B}) connectable to said catheter means (C_{A}, C_{B}) and configured to aspirate an amount of said immiscible fluid (AB) into said first catheter means (C_{A}) and to move said amount of said immiscible fluid (AB) to said three-way junction (C_{J}) and arrange a first part (AB₁) of said immiscible fluid (AB) in a part of said second catheter means (C_{B}) and a second part (AB₂) of said immiscible fluid in a part of said first catheter means (C_{A}); whereby said first (AB₁) and second (AB₂) parts of said immiscible fluid (AB) being configured to separate a taken sample (TS) from the rinsing fluid, the system further comprising a source of a rinsing fluid (F_{A}, F_{B}) connectable to said catheter means (C_{A}, C_{B}) and configured to supply a rinsing fluid from said source (F_{A}, F_{B}) to said catheter means (C_{A}, C_{B}) wherin the pumping means (P_{A}, P_{B}) are configured to provide a flow of rinsing fluid from said source (F_{A}, F_{B}) of rinsing fluid through said catheter means (C_{A}, C_{B}) to a waste tube at the delivery end (C_{DE}) of said catheter means (C_{B}), **characterized in that** the flow of rinsing fluid is accomplished by means of a first pumping means (P_{A}) providing a pushing action equal to a suction action provided by a second pumping means (P_{B}), whereby the rinsing fluid will pass by said sample-taking end (CE) without entering it when flowing from said first catheter means (C_{A}) to said second catheter means (C_{B}).

2. The system as recited in claim 1, wherein the flow of rinsing fluid is accomplished by means of a first pumping means (P_{A}) pushing with a slightly higher pressure than a second pumping means (P_{B}) is sucking, whereby a part of the rinsing fluid enters and rinses said sample-taking end (C_{TE}) of the catheter means (C_{A}, C_{B}).

3. The system as recited in claim 2, wherein said sample-taking end (C_{TE}) is rinsed by means of said first pumping means (P_{A}) pushing at 100% of a flow F and said second pumping means (P_{B}) sucking at 90% of said flow F.

4. The system as recited in any of the preceding claims, wherein said pumping means (P_{A}, P_{B}) is configured as one single double-acting suction and force pumping means with a first part (P_{A}) having the capability of providing a pushing action and a second part (P_{B}) having the capability of providing a suction action, or vice versa, and wherein said first and second parts (P_{A}, P_{B}) further being configured to operate simultaneously or separately.

5. The system as recited in claim 4, further comprising a third pumping means configured to operate when the first and second parts of said double-acting suction and force pumping means (P_{A}, P_{B}) are operated separately and to compensate for the action of the active one of said first (P_{A}) and second parts (P_{B}).

6. The system as recited in any of the preceding claims, further comprising analysing means (AM) configured to analyse said taken fluid sample (TS).

7. The system as recited in any of the preceding claims, further comprising a source of a drug solution connectable to said catheter means (C_{A}, C_{B}), said pumping means (P_{A}, P_{B}) being configured to transport an amount of said drug to said sample-taking end (C_{TE}) and supply said a drug to said sample site (SS).

8. The system as recited in any of the preceding claims where the immiscible fluid is air.

9. A method for automatic taking of a fluid sample from a sample site (SS) of a living test object, comprising the steps of:
- supplying a rinsing fluid to a catheter means (CA, CB)
- aspirating an amount of an immiscible fluid (AB) into the catheter means (CA, CB) ;
- moving said amount of said immiscible fluid (AB) to a three-way junction (CJ) of said catheter means (CA, CB), by controlling a first pumping means (PA) connected to first catheter means (CA) to provide a pushing action and a second pumping means (PB) connected to second catheter means (CB) to provide a suction action, the pushing and suction actions being of the same size or almost of the same size;
- moving a first part (AB1) of said immiscible fluid (AB) towards an opening of a sample-taking end (CTE);
- withdrawing a fluid sample (TS);
- arranging a second part (AB2) of said immiscible fluid (AB) after said taken sample (TS);
- moving said taken sample (TS) in said catheter means (CA, CB) to a sample-delivery end (CDE) at a sample tube (T); delivering said taken sample (TS) to said sample tube (T); and
- rinsing the lumens of said catheter means (CA, CB) by providing a flow of rinsing fluid through said catheter means (CA, CB).

10. A computer program product for use in an automatic system for taking of a fluid sample from a sample site (SS) of a living test object, said computer program product comprising computer code portions configured to realise the method steps of claim 9.

## Patentansprüche

1. Automatisches System (10) zur Entnahme einer Flüssigkeitsprobe aus einer Probenstelle (SS) eines lebenden Testobjekts, das aufweist:
- Kathetereinrichtungen (C_{A}, C_{B}), die eine Dreiwegverbindung (CJ) aufweisen, die gestaltet ist, in der Nähe der Probenstelle (SS) angeordnet zu werden, wobei die Dreiwegverbindung (CJ) mit einer ersten Kathetereinrichtung (C_{A}) , einer zweiten Kathetereinrichtung (C_{B}) und einem Probenentnahmeende (C_{TE}) verbunden ist;
- ein Ventil (V_{A2}), das mit der ersten Kathetereinrichtung (C_{A}) verbunden ist, wobei das Ventil (V_{A2}) einen Einlaß (V_{I}) für ein unvermischbares Fluid aufweist, das in die erste Kathetereinrichtung (C_{A}) eingesaugt werden soll; und
- Pumpeinrichtungen (P_{A}, P_{B}), die mit den Kathetereinrichtungen (C_{A}, C_{B}) verbindbar sind und gestaltet sind, eine Menge des unvermischbaren Fluids (AB) in die erste Kathetereinrichtung (C_{A}) einzusaugen und die Menge des unvermischbaren Fluids (AB) zur Dreiwegverbindung (CJ) zu bewegen und einen ersten Teil (AB₁) des unvermischbaren Fluids (AB) in einem Teil der zweiten Kathetereinrichtung (C_{B}) anzuordnen und einen zweiten Teil (AB₂) des unvermischbaren Fluids in einem Teil der ersten Kathetereinrichtung (C_{A}) anzuordnen; wodurch der erste (AB₁) und zweite (AB₂) Teil des unvermischbaren Fluids (AB) gestaltet ist, eine entnommene Probe (TS) aus der Spülflüssigkeit abzutrennen, wobei das System ferner eine Quelle einer Spülflüssigkeit (F_{A}, F_{B}) aufweist, die mit den Kathetereinrichtungen (C_{A}, C_{B}) verbindbar ist und gestaltet ist, den Kathetereinrichtungen (C_{A}, C_{B}) eine Spülflüssigkeit aus der Quelle (F_{A}, F_{B}) zuzuführen, wobei die Pumpeinrichtungen (P_{A}, P_{B}) gestaltet sind, einen Spülflüssigkeitsfluß aus der Quelle (F_{A}, F_{B}) der Spülflüssigkeit durch die Kathetereinrichtungen (C_{A}, C_{B}) zu einer Ablaufröhre am Abgabeende (CDE) der Kathetereinrichtungen (C_{B}) zu liefern, **dadurch gekennzeichnet, daß** der Fluß der Spülflüssigkeit mittels einer ersten Pumpeinrichtung (P_{A}) erreicht wird, die eine Schubwirkung bereitstellt, die gleich einer Saugwirkung ist, die durch eine zweite Pumpeinrichtung (P_{B}) bereitgestellt wird, wodurch die Spülflüssigkeit durch das Probenentnahmeende (CE) gehen wird, ohne in es einzutreten, wenn es aus der ersten Kathetereinrichtung (C_{A}) zur zweiten Kathetereinrichtung (C_{B}) fließt.

2. System nach Anspruch 1, wobei der Spülflüssigkeitsfluß mittels einer ersten Pumpeinrichtung (P_{A}) erreicht wird, die mit einem geringfügig höheren Druck schiebt als eine zweite Pumpeinrichtung (P_{B}) saugt, wodurch ein Teil der Spülflüssigkeit in das Probenentnahmeende (C_{TE}) der Kathetereinrichtungen (C_{A}, C_{B}) eintritt und sie spült.

3. System nach Anspruch 2, wobei das Probenentnahmeende (C_{TE}) mittels der ersten Pumpeinrichtung (P_{A}), die mit 100% eines Flusses F schiebt, und der zweiten Pumpeinrichtung (P_{B}), die mit 90% des Flusses F saugt, gespült wird.

4. System nach einem der vorhergehenden Ansprüche, wobei die Pumpeinrichtungen (P_{A}, P_{B}) als eine einzige doppeltwirkende Saug- und Druckpumpeinrichtung mit einem ersten Teil (P_{A}), der die Fähigkeit aufweist, eine Schubwirkung bereitzustellen, und einem zweiten Teil (P_{B}), der die Fähigkeit aufweist, eine Saugwirkung bereitzustellen, oder umgekehrt gestaltet sind, und wobei die ersten und zweiten Teile (P_{A}, P_{B}) ferner gestaltet sind, gleichzeitig oder getrennt zu arbeiten.

5. System nach Anspruch 4, das ferner eine dritte Pumpeinrichtung aufweist, die gestaltet ist zu arbeiten, wenn der ersten und zweite Teil der doppeltwirkenden Saug- und Druckpumpeinrichtung (P_{A}, P_{B}) getrennt betrieben wird und die Wirkung des aktiven des ersten (P_{A}) und zweiten Teils (P_{B}) zu kompensieren.

6. System nach einem der vorhergehenden Ansprüche, das ferner eine Analyseeinrichtung (AM) aufweist, die gestaltet ist, die entnommene Flüssigkeitsprobe (TS) zu analysieren.

7. System nach einem der vorhergehenden Ansprüche, das ferner eine Quelle einer Arzneilösung aufweist, die mit den Kathetereinrichtungen (C_{A}, C_{B}) verbindbar ist, wobei die Pumpeinrichtungen (P_{A}, P_{B}) gestaltet sind, eine Menge der Arznei zum Probenentnahmeende (C_{TE}) zu transportieren und die Arznei der Probenstelle (SS) zuzuführen.

8. System nach einem der vorhergehenden Ansprüche, wobei das unvermischbare Fluid Luft ist.

9. Verfahren zum automatischen Entnehmen einer Flüssigkeitsprobe aus einer Probenstelle (SS) eines lebenden Testobjekts, das die Schritte aufweist:
- Zuführen einer Spülflüssigkeit zu Kathetereinrichtungen (C_{A}, C_{B}) ;
- Einsaugen einer Menge eines unvermischbaren Fluids (AB) in die Kathetereinrichtungen (C_{A}, C_{B});
- Bewegen der Menge des unvermischbaren Fluids (AB) zu einer Dreiwegverbindung (CJ) der Kathetereinrichtungen (C_{A}, C_{B}), indem eine erste Pumpeinrichtung (P_{A}), die mit einer ersten Kathetereinrichtung (C_{A}) verbunden ist, so gesteuert wird, daß sie eine Schubwirkung bereitstellt, und eine zweite Pumpeinrichtung (P_{B}), die mit einer zweiten Kathetereinrichtung (C_{B}) verbunden ist, so gesteuert wird, daß sie eine Saugwirkung bereitstellt, wobei die Schub- und Saugwirkungen dieselbe Größe oder fast dieselbe Größe aufweisen;
- Bewegen eines ersten Teils (AB₁) des unvermischbaren Fluids (AB) zu einer Öffnung einer Probenentnahmeende (C_{TE}) ;
- Entnehmen einer Flüssigkeitsprobe (TS)
- Anordnen eines zweiten Teils (AB₂) des unvermischbaren Fluids (AB) nach der entnommenen Probe (TS);
- Bewegen der entnommenen Probe (TS) in den Kathetereinrichtungen (C_{A}, C_{B}) zu einem Probenabgabenende (CDE) an einer Probenröhre (T);
- Abgeben der entnommenen Probe (TS) an die Probenröhre (T); und
- Spülen der Lumina der Kathetereinrichtungen (C_{A}, C_{B}) durch Bereitstellen eines Spülflüssigkeitsflusses durch die Kathetereinrichtungen (C_{A}, C_{B}).

10. Computerprogrammprodukt zur Verwendung in einem automatischen System zur Entnahme einer Flüssigkeitsprobe aus einer Probenstelle (SS) eines lebenden Testobjekts, wobei das Computerprogrammprodukt Computercodeabschnitte aufweist, die gestaltet sind, die Verfahrensschritte des Anspruchs 9 zu verwirklichen.

## Revendications

1. Système automatique (10) pour prélever un échantillon de fluide à partir d'un site d'échantillon (SS) d'un objet d'essai vivant, comprenant :
des moyens de cathéter (C_{A}, C_{B}) comprenant une jonction à trois voies (C_{J}) configurée pour être située à proximité dudit site d'échantillon (SS), ladite jonction à trois voies (C_{J}) étant raccordée à un premier moyen de cathéter (C_{A}), à un deuxième moyen de cathéter (C_{B}) et à une extrémité de prélèvement d'échantillon (C_{TE}) ;
une vanne (V_{A2}) raccordée audit premier moyen de cathéter (C_{A}), ladite vanne (V_{A2}) ayant une entrée (V_{I}) pour un fluide immiscible destiné à être aspiré dans ledit premier moyen de cathéter (C_{A}) ; et
des moyens de pompage (P_{A}, P_{B}) raccordables auxdits moyens de cathéter (C_{A}, C_{B}) et configurés pour aspirer une quantité dudit fluide immiscible (AB) dans ledit premier moyen de cathéter (C_{A}) et pour envoyer ladite quantité dudit fluide immiscible (AB) vers ladite jonction à trois voies (C_{J}) et pour agencer une première partie (AB₁) dudit fluide immiscible (AB) dans une partie dudit deuxième moyen de cathéter (C_{B}) et une deuxième partie (AB₂) dudit fluide immiscible dans une partie dudit premier moyen de cathéter (C_{A}) ; grâce à quoi lesdites première (AB₁) et deuxième (AB₂) parties dudit fluide immiscible (AB) sont configurées pour séparer un échantillon prélevé (TS) du fluide de rinçage, le système comprenant en outre une source d'un fluide de rinçage (F_{A}, F_{B}) raccordable auxdits moyens de cathéter (C_{A}, C_{B}) et configurée pour envoyer un fluide de rinçage provenant de ladite source (F_{A}, F_{B}) vers lesdits moyens de cathéter (C_{A}, C_{B}) où les moyens de pompage (P_{A}, P_{B}) sont configurés pour fournir un écoulement de fluide de rinçage provenant de ladite source (F_{A}, F_{B}) de fluide de rinçage par lesdits moyens de cathéter (C_{A}, C_{B}) à un tube d'évacuation à l'extrémité de distribution (C_{DE}) dudit moyen de cathéter (C_{B}), **caractérisé en ce que** l'écoulement du liquide rinçage est accompli au moyen d'un premier moyen de pompage (P_{A}) fournissant une action de poussée égale à une action d'aspiration fournie par un deuxième moyen de pompage (P_{B}), grâce à quoi le fluide rinçage passe par ladite extrémité de prélèvement d'échantillon (CE) sans y entrer lorsque le fluide s'écoule à partir dudit premier moyen de cathéter (C_{A}) audit deuxième moyen de cathéter (C_{B}).

2. Système selon la revendication 1, dans lequel l'écoulement du fluide de rinçage est accompli au moyen d'un premier moyen de pompage (P_{A}) exerçant une poussée avec une pression légèrement supérieure à l'aspiration d'un deuxième moyen de pompage (P_{B}), grâce à quoi une partie du fluide de rinçage entre et rince ladite extrémité de prélèvement d'échantillon (C_{TE}) des moyens de cathéter (C_{A}, C_{B}).

3. Système selon la revendication 2, dans lequel ladite extrémité de prélèvement d'échantillon (C_{TE}) est rincée au moyen dudit premier moyen de pompage (P_{A}) exerçant une poussée à 100 % d'un écoulement F et dudit deuxième moyen de pompage (P_{B}) aspirant à 90 % dudit écoulement F.

4. Système selon l'une quelconque des revendications précédentes, dans lequel lesdits moyens de pompage (P_{A}, P_{B}) sont configurés en tant qu'une seule aspiration à double action et des moyens de force de pompage avec une première partie (P_{A}) ayant la capacité de fournir une action de poussée et une deuxième partie (P_{B}) ayant la capacité de fournir une action d'aspiration, ou vice et versa, et où lesdites première et deuxième parties (P_{A}, P_{B}) sont configurées en outre pour fonctionner simultanément ou séparément.

5. Système selon la revendication 4, comprenant en outre un troisième moyen de pompage configuré pour fonctionner avec les première et deuxième parties de ladite aspiration à double action et des moyens de force de pompage (P_{A}, P_{B}) sont actionnés séparément et compensent l'action d'une partie active desdites première (P_{A}) et deuxième parties (P_{B}).

6. Système selon l'une quelconque des revendications précédentes, comprenant en outre des moyens d'analyse (AM) configurés pour analyser ledit échantillon de fluide prélevé (TS).

7. Système selon l'une quelconque des revendications précédentes, comprenant en outre une source d'une solution médicamenteuse raccordable auxdits moyens de cathéter (C_{A}, C_{B}), lesdits moyens de pompage (P_{A}, P_{B}) étant configurés pour transporter une quantité dudit médicament à ladite extrémité de prélèvement d'échantillon (C_{TE}) et pour fournir ledit médicament audit site d'échantillon (SS).

8. Système selon l'une quelconque des revendications précédentes dans lequel le fluide immiscible est l'air.

9. Procédé pour le prélèvement automatique d'un échantillon de fluide à partir d'un site d'échantillon (SS) d'un objet d'essai vivant, comprenant les étapes consistant à :
envoyer un fluide de rinçage vers des moyens de cathéter (CA, CB) ;
aspirer une quantité d'un fluide immiscible (AB) dans le moyen de cathéter (CA, CB) ;
déplacer ladite quantité de fluide immiscible (AB) vers une jonction à trois voies (C_{J}) desdits moyens de cathéter (CA, CB), en commandant un premier moyen de pompage (PA) raccordé au premier moyen de cathéter (CA) pour fournir une action de poussée et un deuxième moyen de pompage (PB) raccordé à un deuxième moyen de cathéter (CB) pour fournir une action d'aspiration, les actions de poussée et d'aspiration étant de même taille ou quasiment de même taille ;
déplacer une première partie (AB₁) dudit fluide immiscible (AB) vers une ouverture d'une extrémité de prélèvement d'échantillon (CTE) ;
recueillir un échantillon de fluide (TS) ;
agencer une deuxième partie (AB2) dudit fluide immiscible (AB) après ledit échantillon prélevé (TS) ;
déplacer ledit échantillon prélevé (TS) dans lesdits moyens de cathéter (CA, CB) à une extrémité de distribution d'échantillon (CDE) à un tube échantillon (T) ;
distribuer ledit échantillon prélevé (TS) audit tube échantillon (T) ; et
rincer les lumières desdits moyens de cathéter (CA, CB) en fournissant un écoulement de fluide de rinçage par lesdits moyens de cathéter (CA, CB).

10. Produit de programme informatique pour une utilisation dans un système automatique pour prélever un échantillon de fluide à partir d'un site d'échantillon (SS) d'un objet d'essai vivant, ledit produit de programme informatique comprenant des parties de code informatique configurées pour réaliser les étapes de procédé de la revendication 9.
